(19)

Europäisches
Patentamt
European
Patent Office
Office européen
des brevets

(11) **EP 1 980 627 A1**

(12) **EUROPEAN PATENT APPLICATION**

(43) Date of publication:
**15.10.2008 Bulletin 2008/42**

(51) Int Cl.:
*C12Q 1/68* [(2006.01)]     *G01N 33/53* [(2006.01)]

(21) Application number: **07106177.4**

(22) Date of filing: **13.04.2007**

| | |
|---|---|
| (84) Designated Contracting States:<br>**AT BE BG CH CY CZ DE DK EE ES FI FR GB GR HU IE IS IT LI LT LU LV MC MT NL PL PT RO SE SI SK TR**<br>Designated Extension States:<br>**AL BA HR MK RS** | (72) Inventors:<br>• **Rosell Costa, Rafael**<br>  **08916 Badalona (ES)**<br>• **Tarón Roca, Miguel**<br>  **08916 Badalona (ES)** |
| (71) Applicant: **Pangaea Biotech, S.A.**<br>**08028 Barcelona (ES)** | (74) Representative: **ABG Patentes, S.L.**<br>**Avenida de Burgos 16D**<br>**Edificio Euromor**<br>**28036 Madrid (ES)** |

(54) **Method of determining a chemotherapeutic regime and survival expectancy for non small cell lung cancer based on EGFR/CSF-1/CA IX expression**

(57)     The present invention relates to a screening method for classifying and for deciding to apply chemotherapy to patients suffering from non-small-cell lung cancer (NSCLC), based on detecting the levels of EGFR, CSF-1 and CA IX.

EP 1 980 627 A1

**Description**

**FIELD OF THE INVENTION**

The present invention relates to the use of EGFR, CSF-1 and/or CA IX as prognostic markers for survival of patients suffering from non small cell lung carcinoma (NSCLC) as well as for deciding whether to apply chemotherapy to NSCLC patients.

**BACKGROUND OF THE INVENTION**

Non-small-cell lung cancer (NSCLC) accounts for approximately 80% of all lung cancers, with 1.2 million new cases worldwide each year. NSCLC resulted in more than one million deaths worldwide in 2001 and is the leading cause of cancer-related mortality in both men and women (31% and 25%, respectively).

NSCLC comprises a group of heterogeneous diseases grouped together because their prognosis and management is roughly identical. However, the following subtypes based on their histology can be identified: (i) *Squamous cell carcinoma* (SCC), accounting for 30% to 40% of NSCLC, also starts in the larger breathing tubes but grows slower meaning that the size of these tumors varies on diagnosis, (ii) *Adenocarcinoma* is the most common subtype of NSCLC, accounting for 50% to 60% of NSCLC, which starts near the gas-exchanging surface of the lung and which includes a subtype, the *bronchioalveolar carcinoma,* which may have different responses to treatment and (iii) *large cell carcinoma* is a fast-growing fonn that grows near the surface of the lung. It is primarily a diagnosis of exclusion, and when more investigation is done, it is usually reclassified to squamous cell carcinoma or adenocarcinoma.

Stage grouping of the NSCLC patients in TNM subsets (T = primary tumour; N = regional lymph nodes; M = distant metastases) permits the identification of patient groups with similar prognosis and treatment options. The stages are defined as follows:

Stage I: Cancer is located in only one lung and has not spread to the adjacent lymph nodes or outside the chest.

Stage II: Cancer is located in one lung and may involve lymph nodes on the same side of the chest but does not include lymph nodes in the space between the lungs (the mediastinum) or outside the chest.

Stage IIIA: Cancer is a single tumour or mass that is not invading any adjacent organs and involves one or more lymph nodes away from the tumour, but not outside the chest.

Stage IIIB: Cancer has spread to more than one area in the chest, but not outside the chest.

Stage IV: Cancer has spread, or metastasized, to different sites in the body, which may include the liver, brain or other organs.

The prognosis of advanced NSCLC is dismal. A recent Eastern Cooperative Oncology Group trial of 1155 patients showed no differences among the chemotherapies used: cisplatin/paclitaxel, cisplatin/gemcitabine, cisplatin/docetaxel and carboplatin/paclitaxel. Overall median time to progression was 3,6 months, and median survival was 7,9 months.

The overall five-year survival of patients with NSCLC has remained at less than 15% for the past 20 years. However, the five-year survival varies according to the TNM subset of the patient, being around 60% for pathologic stage IA and IB, 34% for pathological stage IIB (T1-2N1M0, T3N0M0), 13% for stage IIIA (T3N1M0, T1-2-3N2M0), and a low 7% for stage IIIB (T4N0-1-2M0).

In stage I and stage II NSCLC patients, approximately 40% of stage I patients and 66% of stage II NSCLC patients die within five years of surgery, mainly due to the development of distant metastases. This group comprises patients who might derive a notable benefit from adjuvant chemotherapy. Unfortunately, there is at present no reliable clinical predictor of recurrence available.

During the past 30 years medical oncologists have focused to optimise the outcome of cancer patients and it is just now that the new technologies available are allowing to investigate polymorphisms, gene expression levels and gene mutations aimed to predict the outcome and the impact of a given therapy in different groups of cancer patients to tailor chemotherapy. Representative examples include the relation between the TS mRNA expression and the response and the survival with antifolates (see EP 1 381 691), beta tubulin III mRNA levels and response to tubulin interacting agents, PTEN methylation and resistance to CPT-11 and STAT3 over expression and resistance to EGF interacting agents.

Although a wealth of data indicates that changes in the level of several gene transcripts can influence the survival expectancy of and the differential chemosensitivity between NSCLC patients with the same TNM subset, at present no predictive genetic markers of chemotherapy response are used for tailoring treatment. To further improve the survival rate in patients with NSCLC, their prognostic classification based on molecular alterations is crucial. Such classification will provide more accurate and useful diagnostic tools and, eventually, more effective therapeutic options.

The epithelial growth factor receptor (EGFR) is the receptor for epidermal growth factor (EGF). It is a member of the ErbB family of receptors, a subfamily of four closely related receptor tyrosine kinases: EGFR (ErbB-1), HER2/c-neu (ErbB-2), Her 3 (ErbB-3) and Her 4 (ErbB-4). EGFR exists on the cell surface and is activated by binding of its specific ligands, including epidermal growth factor and transforming growth factor $\alpha$ (TGF$\alpha$). Upon activation by its growth factor ligands, EGFR undergoes a transition from an inactive monomeric form to an active homodimer. EGFR dimerization stimulates its intrinsic intracellular protein-tyrosine kinase activity. As a result, autophosphorylation of five tyrosine residues in the C-terminal domain of EGFR occurs. This autophosphorylation elicits downstream activation and signaling by several other proteins that associate with the phosphorylated tyrosines through their own phosphotyrosine-binding SH2 domains. These downstream signaling proteins initiate several signal transduction cascades, principally the MAPK, Akt and JNK pathways, leading to DNA synthesis and cell proliferation. Such proteins modulate phenotypes such as cell migration, adhesion, and proliferation. The kinase domain of EGFR can also cross-phosphorylate tyrosine residues of other receptors it is aggregated with, and can itself be activated in that manner.

Mutations that lead to EGFR overexpression (known as upregulation) or overactivity have been associated with a number of cancers, including glioblastoma multiforme and lung cancer. In particular, Jeon Y-K. et al (Lung Cancer. 2006 Dec;54(3):387-98) have shown a correlation between EGFR gene expression levels and a reduction in the survival rates in SCC, especially stage I SCC. Moreover, EGFR amplification was associated with a shorter survival in node-positive SCC. Hirsch, F.R. et al. (J Clin Oncol. 2003 Oct 15;21(20):3798-807) have also reported that EGFR protein overexpression can be observed in 62% of the NSCLC, more frequently in SCC than non-SCC.

CSF-1, also known as macrophage colony stimulating factor (CSF-1), is responsible for survival and chemotaxis of monocytes. Chen JJ, et al. (J Clin Oncol. 2005, 23:953-64) describes that the overexpression of CSF-1 in many cancers, including NSCLC, coincides with dense infiltration with tumour-associated macrophages. CSF-1 was identified by microarray to be upregulated more than twofold in lung cancer cells after co-culture with macrophages (Uemura,Y. et al. 2004, Int.J.Cancer, 109:826-832). Mroczko et al. (Pol Arch Med Wewn. 2001 Mar;105(3):203-9) has described the appearance of increased CSF-1 levels in the serum from NSCLC patients relative to the control group.

Carbonic anhydrase IX (CA IX), which catalyzes the rapid interconversion of carbon dioxide and water into carbonic acid, protons, and bicarbonate ions, belongs to the group of the membrane associated CAs of the CA alpha family. In hypoxic conditions, hypoxia-inducible factor 1 alpha (HIF-1 alpha) acts as a master transcription regulator for adaptive response, including the upregulation of CA IX. It has been described that low *CA IX* expression is a strong predictor of worse survival in patients with advanced renal cell carcinoma (Bui et al. 2003, Clin Cancer Res 9:802-11). In another study, however, *CA IX* mRNA levels were not demonstrated to bear prognostic value in early-stage NSCLC (Simi, L. et al, Lung Cancer. 2006 Apr; 52(1):59-66) whereas a second immunohistochemical study reported a correlation between high expression and poor outcome (Kon-no, H. et al. Lung Cancer. 2006 Dec; 54(3):409-18). Apart from renal and lung cancer, a relationship between low *CA IX* expression (mRNA) and poor prognosis was also observed by Greiner J et al. (Blood, 2006, 108:4109-17) in other tumours. It seems that the overall expression of *CA IX* decreases with progression and the development of metastases, raising the hypothesis that in the later stages of tumour growth, continued *CA IX* expression is no longer a requirement (Bui, M.H. et al. 2003, Clin.Cancer Res. 9:802-811). Otherwise, high expression of *CA IX* might be viewed as a marker of tumour inadequacy to survive in hypoxic conditions - perhaps as a marker of hypoxia-induced apoptosis.

The reliability of the prognostic methods can be increased by simultaneously measuring the expression of several genes whose expression levels correlate with survival among patients with NSCLC. So far, only few studies have addressed the prognostic value of gene expression profiles in NSCLC (Raponi,M. et al. 2006, Cancer Res. 66: 7466-7472, Chen,J.J. et al. 2005, J.Clin.Oncol. 23:953-964, Inamura,K. et al., 2005, Oncogene, 24:7105-7113, Potti, A. et al. 2006, N.Engl.J.Med., 355:570-580). Simultaneous detection of the expresion of a collection of genes can be done by using cDNA microarrays like, for instance, Chen et al (NEJM, 2007, 356:11-19), who describe a five-gene signature which correlates with clinical outcome of NSCLC.

Reverse-transcriptase polymerase chain reaction (RT-PCR) is also useful for identifying genes that predict survival in NSCLC, it allows for accurate and reproducible RNA quantification and is cheaper and less laborious than cDNA microarrays and can be performed in paraffin-embedded tumour tissue. It has been described by Endoh (J Clin Oncol. 2004, 22:811-9) that the expression levels of 8 genes by RT-PCR correlated with survival in lung adenocarcinoma. Furthermore, a recently identified five-gene signature (RT-PCR) - including genes engaged in tumour-macrophages interaction related to tumor progression - was also able to predict survival in NSCLC (Chen et al., J Clin Oncol. 2005,23: 953-64). When 56 receptor tyrosine kinases (RTKs) were examined by RT-PCR in early-stage NSCLC, the hazard risk for metastasis development was increased in tumours overexpressing 8 of them - including EGFR, NTRK1 and INR1 (Muller-Tidow, C et al. , 2005, Cancer Res 65:1778-82). However, none of these studies have focused on predicting survival of patients suffering from the early stages of NSCLC.

Accordingly, there remains a need in the art for reliable markers which can be used as predictive markers for the outcome of NSCLC patients, in particular in patients of the early stages of NSCLC and which, accordingly, can be used to decide on whether the patients are candidates for chemotherapy or not.

## SUMMARY OF THE INVENTION

It is an object of the invention to provide a tool for use to predicting the prognosis of patients of NSCLC as well as for deciding whether to apply chemotherapy in NSCLC. We have found that EGFR, CSF-1 and CA IX are independent prognostic factors and that the expression values of the three of them can be combined to obtain a risk score which also provides a significant prognostic value for patient survival. Therefore, it can be used for deciding whether to apply chemotherapy, providing an important tool for customizing NSCLC chemotherapy in order to improve survival in this very common and fatal disease.

Accordingly, in a first aspect the invention provides a method for predicting the survival of a patient suffering from non-small-cell lung cancer (NSCLC) comprising the steps of:

> a) isolating mRNA from a tissue, blood or plasma, sample of the patient;
> b) determining the gene expression levels of EGFR, CSF-1 and CA IX genes in the sample;
> c) comparing the expression levels of EGFR, CSF-1 and CA IX genes in the sample with reference values for EGFR, CSF-1 and CA IX expression levels

wherein an increased expression of the EGFR and CSF-1 genes and a decreased expression of the CA IX gene with respect to the reference values is indicative that the survival prognosis of said patient will be decreased with respect to patients wherein expression of the EGFR, CSF-1 and CA IX gene is not altered.

In a further embodiment, the invention comprises a method wherein, in addition to determining the gene expression levels of EGFR, CSF-1 and CA IX genes in a sample, the expression level of one or more additional genes selected from the group of fibronectin 1 fibronectin 1 (*FN1*), insulin receptor 1 *(ISNR),* anillin *(ANLN),* prolyl hydrolase 4 (*PH4*), *VEGF-C, NTRK1* or *KIAA0974* with respect to a reference value is also measured, wherein an increased expression of any of said genes in combination with an increased expression EGFR, CSF-1 and a decreased expression of CA IX is indicative that the survival prognosis of said patient will be decreased with respect to patients wherein expression of the same genes is not altered.

In a second aspect, the invention provides a method for predicting the survival of a patient suffering from non-small-cell lung cancer (NSCLC) comprising the steps of

> a) isolating mRNA from a tissue, blood or plasma sample of the patient;
> b) determining the gene expression level of EGFR, CSF-1 and CA IX genes in the sample;
> (c) calculating a risk score on the basis of the expression levels of EGFR, CSF-1 and CA IX genes using the formula

$$[0,93 \mathrm{x CSF} + 1,4 \mathrm{x CA\ IX} + 1,1\ \mathrm{x\ EGFR}]$$ wherein CSF, CA IX and EGFR are the z-scores of the expression levels of the CSF-1, CA IX and EGFR genes respectively

wherein a risk score higher than 0 is indicative of poor survival.

In another embodiment, the method of the invention is applied to patients wherein the NSCLC is squamous cell carcinoma.

In another embodiment, the method of the invention is applied to stage IA, IB or stage II NSCLC patients.

In yet another embodiment, the nucleic acid is isolated from a tumour sample, from a blood or from a serum sample of the patient.

In a third aspect, the invention provides a method for deciding whether a patient suffering NSCLC should be treated with chemotherapy which comprises the steps of

> a) isolating mRNA from a tissue, blood or plasma sample of the patient;
> b) determining the gene expression level of EGFR, CSF-1 and CA IX genes in the sample;
> c) comparing the expression levels of EGFR, CSF-1 and CA IX genes in the sample with reference values for EGFR, CSF-1 and CA IX expression levels

wherein an increased expression of the EGFR and CSF-1 genes and a decreased expression of the CA IX gene with respect to the reference values is indicative that the patient should be treated with chemotherapy.

In a further aspect, the invention provides a method for deciding whether a patient suffering NSCLC should be treated with chemotherapy comprising the steps of

a) isolating mRNA from a tissue, blood or plasma sample of the patient;
b) determining the gene expression level of EGFR, CSF-1 and CA IX genes in the sample;
(c) calculating a risk score on the basis of the expression levels of EGFR, CSF-1 and CA IX genes using the formula

$$[0,93 \text{x} CSF + 1,4 \text{x} CA\ IX + 1,1\ \text{x}\ EGFR]$$ wherein CSF, CA IX and EGFR are the z-scores of gene expression levels

wherein a risk score higher than 0 is indicative that the patient should be treated with chemotherapy.

In another embodiment, the method for deciding whether a patient suffering NSCLC should be treated with chemotherapy according to the invention is applied to stage IA, IB or stage II NSCLC patients.

In a fifth aspect, the invention provides a kit for detecting altered expression of the genes EGFR, CSF-1 and CA IX which comprises a first pair of primers which is capable of amplifying a region of EGFR, a second pair of primers which is capable of amplifying a region of CSF-1 and a third pair of primers which is capable of amplifying a region of the CA IX gene.

In a further embodiment, the kit also comprises at least an additional pair of primers capable of amplifying a region of fibronectin 1 (*FN1*), insulin receptor 1 (*ISNR*), anillin (*ANLN*), prolyl hydrolase 4 (*PH4*), *VEGF-C, NTRK1* or *KIAA0974.*

In yet a further embodiment, the kit also comprises a reverse transcriptase.

In a further embodiment, the invention relates to the use of the kit comprising the primers specific for detecting altered expression of the genes EGFR, CSF-1 and CA IX for predicting the survival of a patient suffering from NSCLC and for deciding whether a patient suffering NSCLC should be treated with chemotherapy.

## BRIEF DESCRIPTION OF THE FIGURES

Figure 1. Survival curve of the patients according to the risk score.
Figure 2. Survival curves for patients with tumors of 4 cm and smaller according to their risk score (A) and for patients with tumors larger than 4 cm according to their risk score (B).

## DETAILED DESCRIPTION OF THE INVENTION

The present invention resides in the finding that the amount of EGFR, CSF-1 and CA IX mRNA provides a signature which correlates with prognosis and survival of patients suffering from NSCLC. Accordingly, patients carrying the signature (tumours expressing high levels of EGFR, CSF-1 mRNA and low levels of CA IX mRNA) will have low survival expectancy, whereas patients which do not carry the signature will have higher chances to survive. Moreover, the signature can also be used to predict the development of distant metastasis and to decide whether to apply chemotherapy. Thus, those patients carrying the signature should be considered for adjuvant chemotherapy, whereas patients who do not show the signature are not likely to benefit from the chemotherapy. Thus, the signature derived from the expression levels of EGFR, CSF-1 and CA IX will provide an important parameter for customizing NSCLC chemotherapy.

Accordingly, in a first embodiment, the invention provides a method for predicting the survival of a patient suffering from non-small-cell lung cancer (NSCLC) comprising the steps of:

a) isolating mRNA from a tissue, blood or plasma sample of the patient;
b) determining the gene expression levels of EGFR, CSF-1 and CA IX genes in the sample;
c) comparing the expression levels of EGFR, CSF-1 and CA IX genes in the sample with reference values for EGFR, CSF-1 and CA IX expression levels

wherein an increased expression of the EGFR and CSF-1 genes and a decreased expression of the CA IX gene with respect to the reference values is indicative that the survival prognosis of said patient will be decreased with respect to patients wherein expression of the EGFR, CSF-1 and CA IX gene is not altered.

The present method can be applied to any type of tissue, fluid, blood or plasma from a patient. For predicting patient survival and deciding whether to apply chemotherapy, it is preferable to examine the tumour tissue. In a preferred embodiment, a portion of normal tissue from the patient from which the tumour is obtained is also examined. Preferably this is done prior to the chemotherapy, usually during tumour resection.

In performing the methods of the present invention, tumour cells are preferably isolated from the patient. Tumours or portions thereof are surgically resected from the patient or obtained by routine biopsy. RNA isolated from frozen or fresh samples is extracted from the cells by any of the methods typical in the art, for example, Sambrook, Fischer and Maniatis, Molecular Cloning, a laboratory manual, (2nd ed.), Cold Spring Harbor Laboratory Press, New York, (1989). Preferably, care is taken to avoid degradation of the RNA during the extraction process.

in a particular embodiment, the expression level is determined using RNA obtained from a formalin-fixed, paraffin-embedded tissue sample. Other tissue, blood or plasma samples are envisaged, such as fresh tissue from a biopsy or blood samples depending on their availability.

Fixed and paraffin-embedded tissue samples are preferred because there are broadly used storable or archival tissue samples in the field of Oncology. RNA may be isolated from an archival pathological sample or biopsy sample which is first deparaffinised. An exemplary deparaffinisation method involves washing the paraffinised sample with an organic solvent, such as xylene, for example. Deparaffinised samples can be rehydrated with an aqueous solution of a lower alcohol. Suitable lower alcohols, for example include, methanol, ethanol, propanols, and butanols. Deparaffinised samples may be rehydrated with successive washes with lower alcoholic solutions of decreasing concentration, for example. Alternatively, the sample is simultaneously deparaffinised and rehydrated. The sample is then lysed and RNA is extracted from the sample.

While all techniques of gene expression profiling, as well as proteomics techniques, are suitable for use in performing the foregoing aspects of the invention, the gene expression levels are often determined by reverse transcription polymerase chain reaction (RT-PCR).

The determination of the expression of a target mRNA requires the simultaneous measurement of the expression of a constitutive RNA as endogenous control, i.e. a RNA whose expression is constant across all tumour samples studied. Preferred reference RNAs include the ribosomal 18S RNA, the β-actin mRNA and the GAPDH mRNA. Most preferred is the ribosomal 18S RNA as it exhibits the most stable expression across all tumour samples. The raw expression level is then normalised to the endogenous reference gene. Other methods known in the art can be used, as long as relative levels of EGFR, CSF-1 and CA IX can be assigned to the samples. Levels of mRNA or the corresponding protein can be measured to obtain the relative level of EGFR, CSF-1 and CA IX expression. Standard methods of measurement well known in the art are used, see for example EP 1 381 681 incorporated by reference herein in its entirety.

In a preferred embodiment, the reference value used for determining whether the expression of a gene sample is "increased" or "decreased" corresponds to the median value of expression levels of EGFR, CSF-1 and CA IX measured in a RNA sample obtained by pooling equal amounts of RNA from each of the tumour samples obtained by biopsy from cancer patients previous to the neoadjuvant chemotherapeutic treatment. Once this median value is established, the level of this marker expressed in tumours tissues from patients can be compared with this median value, and thus be assigned a level of "increased" or "decreased." In a particular embodiment, an increase in expression above the reference value of at least 1.1-fold, 1.5-fold, 5-fold, 10-fold, 20-fold, 30-fold, 40-fold, 50-fold, 60-fold, 70-fold, 80-fold, 90-fold, 100-fold or even more compared with the reference value is considered as "increased" expression. In a particular embodiment, a decrease in expression below the reference value of at least 0.9-fold, 0.75-fold, 0.2-fold, 0.1-fold, 0.05-fold, 0.025-fold, 0.02-fold, 0.01-fold, 0.005-fold or even less compared with the reference value is considered as "decreased" expression.

Due to inter-subject variability (e.g. aspects relating to age, race, etc.) it is very difficult (if not practically impossible) to establish absolute reference values for EGFR, CSF-1 and CA IX. Thus, in a particular embodiment, the reference values for "increased" or "decreased" EGFR, CSF-1 and CA IX expression are determined by calculating percentiles by conventional means involving the testing of a group of samples isolated from normal subjects (i.e. people with no diagnosis of NSCLC) for the expression levels of the EGFR, CSF-1 and CA IX. The "increased" levels can then be assigned, preferably, to samples wherein expression levels for the EGFR, CSF-1 and CA IX genes is equal to or in excels of percentile 50 in the normal population, including, for example, expression levels equal to or in excess to percentile 60 in the normal population, equal to or in excess to percentile 70 in the normal population, equal to or in excess to percentile 80 in the normal population, equal to or in excess to percentile 90 in the normal population, and equal to or in excess to percentile 95 in the normal population.

The present method is suitable for predicting the survival of patients suffering from any type of NSCLC, including squamous cell carcinoma (SCC), adenocarcinoma, including the bronchioalveolar carcinoma, the most common subtype of NSCLC, and large cell carcinoma. Moreover, the method is also suitable for predicting the development of distant metastasis. Preferably, the method is also suitable for predicting the survival of NSCLC patients wherein the tumor are surgically resectable, including stage I, stage II, and selected stage III tumours, wherein the tumors of different sizes. More preferably, the method of the invention is suitable for predicting the outcome of patients having tumors smaller than 4 cm and even more preferably for predicting the outcome of patients having tumors in stages IA and IB.

In a further embodiment, the invention comprises measuring in the same biological samples the expression levels of at least one additional gene, wherein said gene is selected from the group of fibronectin 1 (FN1), HIF-1alpha

prolyl hydrolase (PH-4), KIAA0974, the actin binding protein anillin (ANLN), the insulin receptor 1 (INSR), vascular endothelial growth factor C (VEGFC) and the neurotrophic tyrosine kinase, receptor, type 1 (NTRK1).

The invention also comprises a method for predicting the survival of a patient suffering from non-small-cell lung cancer (NSCLC) comprising the steps of

a) isolating mRNA from a tissue, blood or plasma sample of the patient;
b) determining the gene expression level of EGFR, CSF-1 and CA IX genes in the sample;
(c) calculating a risk score on the basis of the expression levels of EGFR, CSF-1 and CA IX genes using the formula

$$[0,93 \times CSF + 1,4 \times CA\ IX + 1,1 \times EGFR]$$ wherein CSF, CA IX and EGFR are the z-scores of the gene expression levels for CSF, CA IX and EGFR, respectively.

wherein a risk score higher than 0 is indicative of poor survival.

The gene signature identified in the present inventions is suitable not only for predicting the survival of a patient suffering from NSCLC, but also allows deciding whether a patient suffering NSCLC should be treated with chemotherapy. Accordingly, in a further embodiment, the invention provides a method for deciding whether a patient suffering NSCLC should be treated with chemotherapy which comprises the steps of

a) isolating mRNA from a tissue, blood or plasma sample of the patient;
b) determining the gene expression level of EGFR, CSF-1 and CA IX genes in the sample;
c) comparing the expression levels of EGFR, CSF-1 and CA IX genes in the sample with reference values for EGFR, CSF-1 and CA IX expression levels

wherein an increased expression of the EGFR and CSF-1 genes and a decreased expression of the CA IX gene with respect to the reference values is indicative that the patient should be treated with chemotherapy.

Moreover, the invention also provides a method for deciding whether a patient suffering NSCLC should be treated with chemotherapy comprising the steps of

a) isolating mRNA from a tissue, blood or plasma sample of the patient;
b) determining the gene expression level of EGFR, CSF-1 and CA IX genes in the sample;
(c) calculating a risk score on the basis of the expression levels of EGFR, CSF-1 and CA IX genes using the formula.

$$[0,93 \times CSF + 1,4 \times CA\ IX + 1,1 \times EGFR]$$ wherein CSF, CA IX and EGFR are the z-scores of the gene expression levels for CSF, CA IX and EGFR, respectively

wherein a risk score higher than 0 is indicative that the patient should be treated with chemotherapy.

By chemotherapy it is understood any treatment with cytotoxic substances aimed to treat cancer. Without wishing to be bound by any limitation, the methods of the invention are suitable for predicting whether the patient should be a considered for chemotherapy. As chemotherapy it is understood any treatment with the chemotherapeutic agents that constitute the standard therapy for NSCLC, including cisplatin or carboplatin, in combination with gemcitabine, paclitaxel, docetaxel, etoposide or vinorelbine.

In a preferred embodiment, cisplatin and carboplatin are usually administered as a combination therapy with etoposide, gemcitabine, paclitaxel, docetaxel or vinorelbine.

The chemotherapy agents above mentioned to be used in the methods of this invention will be administered in doses commonly employed clinically. Such doses will be calculated in the normal fashion, for example on body surface area.

The method of the invention is suitable for deciding whether to apply chemotherapy to patients suffering from any type of NSCLC, including squamous cell carcinoma (SCC), adenocarcinoma, including the bronchioalveolar carcinoma, the most common subtype of NSCLC, and large cell carcinoma. Moreover, the method is also suitable for deciding whether to apply chemotherapy to NSCLC patients wherein the tumours are surgically resectable, including stage I, stage 11, and selected stage III tumours, wherein the tumors of different sizes. More preferably, the method of the invention is suitable for predicting the outcome of patients having tumors smaller than 4 cm and even more preferably for predicting the outcome of patients having tumors in stages IA and IB.

Thus, according to the invention, patients which show increased expression of EGFR and CSF-1 and, at the same time, show decreased expression of CA IX, should be considered as candidates for chemotherapy whereas patients showing normal levels of the three genes will not benefit from the chemotherapy.

In a further aspect, the invention provides a kit for detecting altered expression of the genes EGFR, CSF-1 and CA IX which comprises a first pair of primers which is capable of amplifying a region of EGFR, a second pair of primers which is capable of amplifying a region of CSF-1 and a third pair of primers which is capable of amplifying a region of the CA IX gene.

In a particular embodiment of the invention, the serum or plasma may be utilized directly for identification of the expression levels of the EGFR, CSF-1 and CA IX mRNA expression levels. In another particular embodiment, nucleic acid is extracted from plasma or serum as an initial step of the invention. In such cases, the total RNA extracted from said samples would represent the working material suitable for subsequent amplification.

Once the nucleic acid sample has been obtained, complementary DNA is made from the messenger RNA template present in the sample using dNTPs and an RNA-dependent DNA polymerase (reverse transcriptase) through the process of reverse transcription. The above components are combined with a DNA primer in a reverse transcriptase buffer, preferably for an hour at 37°C. Preferred reverse transcriptase useful for carrying out the synthesis of the cDNA are MMLV reverse transcriptase or its RNase H deficient derivatives, AMV Reverse Transcriptase, and RSV reverse transcriptase.

The single stranded cDNA generated from the original single-stranded mRNA is then amplified. In a particular embodiment, the amplification of the DNA is carried out by means of PCR. The general principles and conditions for amplification and detection of nucleic acids, such as using PCR, are well known for the skilled person in the art. In particular, the Polymerase Chain Reaction (PCR) carried out by the method of the present invention uses an appropriate enzyme which is capable of amplifying the target cDNA. Preferred enzymes used for amplification of the target nucleic acids are thermostable DNA polymerases. These DNA polymerases may be isolated from natural or recombinant sources, by techniques that are well-known in the art. As an alternative to isolation, thermostable DNA polymerases are available commercially. Particularly preferred thennostable DNA polymerases for use in the compositions and methods of the present invention include, but are not limited to, Taq, Tne, Tma, TliNENT™, DEEPVENT™, Pfu, Pwo, Tfi or Tth DNA polymerases, or mutants or derivatives thereof

The amplification requires the use of oligonucleotide primers which are capable of specifically amplify the EGFR, CSF-1 and CA IX target sequences.

The terms "oligonucleotide primers" or "amplification oligonucleotides" are herein used indistinguishably and refer to a polymeric nucleic acid having generally less than 1,000 residues, including those in a size range having a lower limit of about 2 to 5 residues and an upper limit of about 500 to 900 residues. In preferred embodiments, oligonucleotide primers are in a size range having a lower limit of about 5 to about 15 residues and an upper limit of about 100 to 200 residues. More preferably, oligonucleotide primers of the present invention are in a size range having a lower limit of about 10 to about 15 residues and an upper limit of about 17 to 100 residues. Although oligonucleotide primers may be purified from naturally occurring nucleic acids, they are generally synthesized using any of a variety of well known enzymatic or chemical methods.

The term "amplification oligonucleotide" refers to an oligonucleotide that hybridizes to a target nucleic acid, or its complement, and participates in a nucleic acid amplification reaction. Amplification oligonucleotides include primers and promoter primers in which the 3' end of the oligonucleotide is extended enzymatically using another nucleic acid strand as the template. In some embodiments, an amplification oligonucleotide contains at least about 10 contiguous bases, and more preferably about 12 contiguous bases, that are complementary to a region of the target sequence (or its complementary strand). Target-binding bases are preferably at least about 80%, and more preferably about 90% to 100% complementary to the sequence to which it binds. An amplification oligonucleotide is preferably about 10 to about 60 bases long and may include modified nucleotides or base analogues. The terms "amplify" or "amplification" refer to a procedure to produce multiple copies of a target nucleic acid sequence or its complement or fragments thereof (i.e., the amplified product may contain less than the complete target sequence). For example, fragments may be produced by amplifying a portion of the target nucleic acid by using an amplification oligonucleotide which hybridizes to, and initiates polymerization from, an internal position of the target nucleic acid. Known amplification methods include, for example, polymerase chain reaction (PCR) amplification, replicase-mediated amplification, ligase chain reaction (LCR) amplification, strand-displacement amplification (SDA) and transcription-associated or transcription-mediated amplification (TMA). PCR amplification uses DNA polymerase, primers for opposite strands and thermal cycling to synthesize multiple copies ofDNA or cDNA. Replicase mediated amplification uses Qβ-replicase to amplify RNA sequences. LCR amplification uses at least four different oligonucleotides to amplify complementary strands of a target by using cycles of hybridization, ligation, and denaturation. SDA uses a primer that contains a recognition site for a restriction endonuclease and an endonuclease that nicks one strand of a hemimodified DNA duplex that includes the target sequence, followed by a series of primer extension and strand displacement steps. An isothermal strand-displacement amplification method that does not rely on endonuclease nicking is also known. Transcription-associated or transcription-mediated amplification uses a primer that includes a promoter sequence and an RNA polymerase specific for the promoter to produce multiple transcripts from a target sequence, thus amplifying the target sequence.

Preferred embodiments of the present invention amplify the EGFR, CSF-1 and CA IX target sequences using the present amplification oligonucleotides in a polymerase chain reaction (PCR) amplification, In another embodiment, the invention comprises the simultaneous detection of the EGFR, CSF-1 and CA IX target sequences and at least one additional gene. Preferred genes to be detected according to the inventions are those genes mentioned in Table 2. Most preferred are the genes fibronectin 1 (*FN1*), insulin receptor 1 (*ISNR*), anillin (*ANLN*), prolyl hydrolase 4 (*PH4*), *VEGF-C, NTRK1* or *KIAA0974,* which genes which show a positive correlation between alteration of expression level and survival prognosis in the NSCLC patients.

The reverse transcription and the amplification reaction can be carried out sequentially in the so-called "uncoupled RT-PCR" wherein the cDNA is obtained by reverse transcription in an independent step and the cDNA is then in a second separate step amplified. The uncoupled method has the advantage that the reaction conditions for the reverse transcription and the amplification can be properly tailored in each step. In contrast, the so-called "coupled RT-PCR" methods, the reverse transcription and the subsequent amplification are carried out in the same tube, which requires the use of a compromised buffer which is appropriate for the activity of the reverse transcriptase and the DNA polymerase.

The kits of the present invention can be used for predicting the survival of a patient suffering from NSCLC as well as for deciding whether a patient suffering NSCLC should be considered for chemotherapy.

The invention being thus described, practice of the invention is illustrated by the experimental examples provided below. The skilled practitioner will realize that the materials and methods used in the illustrative examples can be modified in various ways.

**EXAMPLES**

**Materials and Methods**

*Patients*

The study group included 66 patients with SqCLC who underwent curative pulmonary resection between 2000 and 2004. Thirty-three patients developed distant metastases after surgery, and the remaining 33 patients were free of distant metastasis after a median follow-up of 37 months (range, 24-64 months). Seven out of 33 patients who developed metastasis after operation had also a local relapse. Twenty seven patients had tumour smaller than 4 cm and the remaining 39 had tumour with larger diameter as shown in Table 1. All consecutive stage I-II patients fulfilling the following criteria were enrolled: complete pulmonary resection, availability of tumor specimen (at least 60% of tumour tissue in the section), and at least a 2-year follow-up for non-relapsed patients. Two stage IIIA patients without distant metastases were also included. Patient characteristics are summarized in Table 1. Surgical procedures included pneumonectomies (18), bilobectomies (11), lobectomies (31) segmentectomies (3) and sleeve-resection of the lobe (3). In all instances, complete mediastinal lymphadenectomy was performed and the surgical margins were free of tumor (R0).

Table 1. Patients characteristics

| Variable | Metastasis group (N=33) | No metastasis group (N=33) |
|---|---|---|
| Age (mean/range) years | 61 (37-75) | 63 (48-76) |
| Sex<br>Women<br>Men | <br>4 (12 %)<br>29 (88 %) | <br>10 (30 %)<br>23 (70 %) |
| Stage<br>IA<br>IB<br>IIB<br>IIIA | <br>3 (9 %)<br>16 (48 %)<br>14 (42 %)<br>0 (0 %) | <br>7 (21 %)<br>16 (48 %)<br>8 (24 %)<br>2 (6 %) |
| Grade<br>G1<br>G2<br>G3 | <br>3 (9 %)<br>21 (63 %)<br>9 (26 %) | <br>4 (12 %)<br>24 (72 %)<br>5 (16 %) |

*Gene expression analysis*

Tumour samples were obtained during surgery as blocks of 1cm$^3$ and snap-frozen in liquid nitrogen. Tissues were stored in -80°C until total RNA was extracted with AllPrep kits (Qiagen). Only tumour samples containing more than 60% of tumour tissue on a microscopic section were eligible for further processing. The concentration of RNA was assessed in Nano-drop™ and the quality of obtained RNA was tested on agarose gel. First-strand cDNA was synthesized from 1 μg of total RNA using the High-Capacity cDNA Archive Kit (Applied Biosystems, Foster City, CA). Quantitative RT-PCR reactions of 29 genes (Table 2) were done using Applied Biosystems TaqMan Low Density Arrays (micro fluidic cards) in an ABI PRISM 7900 HT Sequence Detection System (Applied Biosystems). One channel of a microfluidic card was loaded with a mix of 55 μL TaqMan Universal PCR Master Mix (Applied Biosystems) and 55 μL of a cDNA template corresponding to 100 ng of total RNA.

Relative gene expression values were calculated by the ΔΔCt method[14] using the Sequence Detection System (SDS) 2.1 software (Applied Biosystems). The ΔΔCt method gives the amount of target gene normalized to an endogenous reference gene and relative to a calibrator sample (reference for all samples). We normalized the raw gene expression values according to the expression of ribosomal *18S* RNA as it exhibited the most stable expression across all tumour samples. The normalized expression of each gene was calibrated by its expression in an RNA pool (made by pooling equal amounts of total RNA from each sample) and commercially available Universal Reference Human Total RNA (Stratagen). We provide data calibrated according to tumour RNA pool as all the genes were detectable in this calibrator.

### *Statistical analysis*

We applied a method for categorizing SqCLC tumours as having good or poor prognosis based on the gene expression profile. The method consisted of five steps: (1) the gene expression was coded as 0 (low) or 1 (high) if the gene expression was ranked in the ≤ 50th or > 50th percentile, respectively, of this gene expression in all tumour samples; (2) a univariate Cox model with overall survival as the dependent variable was constructed and categorized with gene expression levels as independent variables; (3) the genes that were significant in the univariate analysis were included in a multivariate Cox proportional-hazards for survival; (4) the risk score for each patient was calculated; (5) in order to find the highest significance level by log-rank test for disease-free survival, different cut-off values for the risk score were tested with the minimum P value method, where the cut-off point is chosen such that the P value for the survival curves comparison is minimal. Data on patients were analyzed from the date of surgery to the time of relapse or death, or the date on which data were censored. Curves for overall survival and disease-free survival were obtained with the Kaplan-Meier product limit method for each prognostic factor and risk group. Comparisons were made with the two-sided log-rank test. Patient characteristics were compared between the two risk groups using the Mann-Whitney test. The chi-square and Fisher's exact tests were used to compare categorical variables. All statistical analyses were carried out at 5% level of significance and with a power of 80%, using the Statistical Package for the Social Sciences, version 13 (SPSS Inc, Chicago, IL).

### Results

Of the 29 genes examined, 10 (CSF1, FN1, CA IX, PH4, KIAA0974, ANLN, VEGFC, ISNR, NTRK1, EGFR) were found to be significantly correlated with survival in the univariate Cox regression analysis, i.e. those genes having a p value lower than 0.05 (values in bold in Table 2).

**Table 2.** Univariate analysis of overall survival

| Gene name/median expression value | Median survival | 95% C.I | p value |
|---|---|---|---|
| CSF1 | | | **0.002** |
| ≤0.90 | NR | - | |
| >0.90 | 27.5 | 20.1-34.9 | |
| FN1 | | | **0.002** |
| ≤0.57 | NR | - | |
| >0.57 | 31.3 | 19.3-43.2 | |
| CA9 | | | **0.007** |
| ≤0.56 | 29 | 20.9-37.2 | |
| >0.56 | NR | | |
| PH4 | | | **0.01** |

(continued)

| Gene name/median expression value | Median survival | 95% C.I | p value |
|---|---|---|---|
| ≤0.90 | NR | - | |
| >0.90 | 29.7 | 21.9-37.5 | |
| KIAA0974 | | | **0.02** |
| ≤0.71 | NR | - | |
| >0.71 | 21.3 | 22.3-40.2 | |
| ANLN | | | **0.02** |
| ≤1.09 | NR | - | |
| >1.09 | 31.3 | 24.4-38.2 | |
| ISNR | | | **0.03** |
| ≤0.57 | NR | - | |
| >0.57 | 33.2 | 19.3-47.1 | |
| VEGFC | | | **0.03** |
| ≤0.67 | NR | - | |
| >0.67 | 33.2 | 26.9-39.5 | |
| NTRK1 | | | **0.04** |
| ≤0.53 | NR | - | |
| >0.53 | 33.2 | 24.9-41.6 | |
| EGFR | | | **0.05** |
| ≤0.76 | NR | - | |
| >0.76 | 33.9 | 23.7-44.2 | |
| SERPINE | | | **0.08** |
| ≤0.45 | 46.9 | | |
| >0.45 | 33.2 | 19.2-46.6 | |
| Ezrin | | | **0.10** |
| ≤0.81 | NR | - | |
| >0.81 | 33.2 | 23.1-43.3 | |
| ARHGDIB | | | **0.11** |
| ≤0.76 | 41.3 | - | |
| >0.76 | 29.7 | 19.8-39.5 | |
| Selectin P ligand | | | 0.12 |
| ≤0.71 | 46.9 | 33.1-60.8 | |
| >0.71 | 29.7 | 20.2-39.2 | |
| CXCR4 | | | 0.18 |
| ≤0.61 | 41.3 | | |
| >0.61 | 31.3 | 25.6-36.9 | |
| STXIA | | | 0.18 |
| ≤0.72 | 46.9 | - | |
| >0.72 | 33.6 | 27.9-39.3 | |

(continued)

| Gene name/median expression value | Median survival | 95% C.I | p value |
|---|---|---|---|
| PDPN | | | 0.19 |
| ≤0.59 | 46.9 | - | |
| >0.59 | 33.2 | 20.6-45.9 | |
| HGF | | | 0.21 |
| ≤0.51 | 46.9 | 32.9-61.1 | |
| >0.51 | 33.2 | 26-40.5 | |
| Selectin L ligand | | | 0.33 |
| ≤0.59 | 41.3 | 30.3-52.3 | |
| >0.59 | 33.2 | 23.1-43.4 | |
| PGK1 | | | 0.35 |
| ≤0.90 | 33.6 | 20.4-46.8 | |
| >0.90 | 38.2 | 28.8-47.5 | |
| IL8 | | | 0.86 |
| ≤0.46 | 36.7 | - | |
| >0.46 | 38.2 | 29.1-47.2 | |
| CD44 | | | 0.42 |
| ≤1.62 | 33.6 | 21.6-45.6 | |
| >1.62 | 46.9 | 31.9-62 | |
| CD47 | | | |
| ≤0.70 | 41.3 | - | 0.36 |
| >0.70 | 33.9 | 17-50.9 | |
| NTRK2 | | | 0.68 |
| ≤0.32 | 41.3 | 28.3-54.3 | |
| >0.32 | 38.2 | - | |
| N-cadherin | | | 0.34 |
| ≤0.34 | 46.9 | - | |
| >0.34 | 33.6 | 27-40 | |
| TWIST1 | | | 0.69 |
| ≤0.77 | 36.7 | 25.8-47.6 | |
| >0.77 | 46.9 | 28.7-65.2 | |
| S100P | | | 0.64 |
| ≤0.21 | 33.6 | 26-41.2 | |
| >0.21 | 46.9 | 34.9-59.1 | |
| GPI | | | 0.52 |
| ≤0.72 | 33.9 | 22.4-45.5 | |
| >0.72 | 46.9 | 25.7-68.2 | |
| MMP9 | | | 0.53 |
| ≤0.51 | 41.3 | 29.9-52.7 | |

(continued)

| Gene name/median expression value | Median survival | 95% C.I | p value |
|---|---|---|---|
| >0.51 | 33.6 | 21.9-45.3 | |

These genes were included in a multivariate Cox regression analysis together with stage, differentiation, tumour size (< 4 cm vs. > 4 cm in diameter), sex and age. Three genes (CSF-1, EGFR, CA IX) and tumour size emerged as independent prognostic factors (Table 3).

**Table 3.** Multivariate Cox model for overall survival.

| | HR | 95% CI | p |
|---|---|---|---|
| Tumor size | | | |
| ≤4 | 1 (ref.) | | |
| >4 | 2.7 | 1.1-6.6 | 0.02 |
| CSF1 | | | |
| ≤0.90 | 1 (ref.) | 1.5-8.5 | 0.005 |
| >0.90 | 3.5 | | |
| EGFR | | | |
| ≤0.76 | 1 (ref.) | | |
| >0.76 | 2.7 | 1.2-6.4 | 0.02 |
| CA9 | | | |
| ≤0.56 | 1 (ref.) | | |
| >0.56 | 0.2 | 0.07-0.43 | <0.0001 |

A risk score was generated by adding the z-scores of gene expression level times its corresponding coefficient as follows: Risk score (RS) = [0.93xCSF + 1.4xCA9 + 1.1xEGFR]. The risk score was used to classify patients into high (RS>0) or low (RS≤0) risk expression profile, in which a high risk score predicted poor survival.

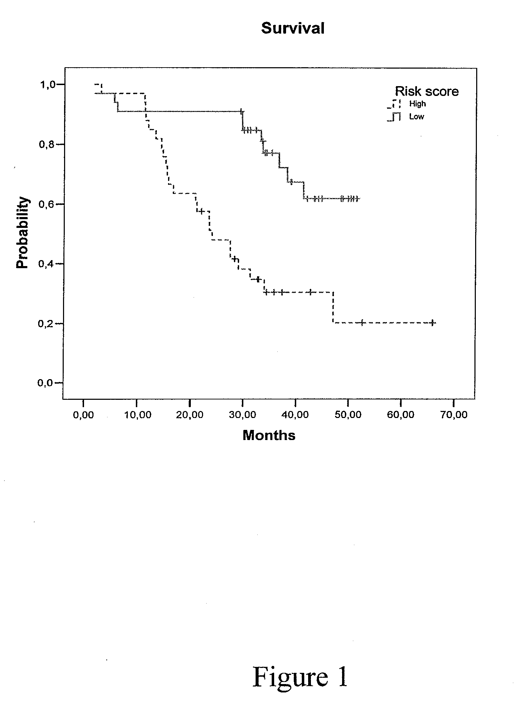

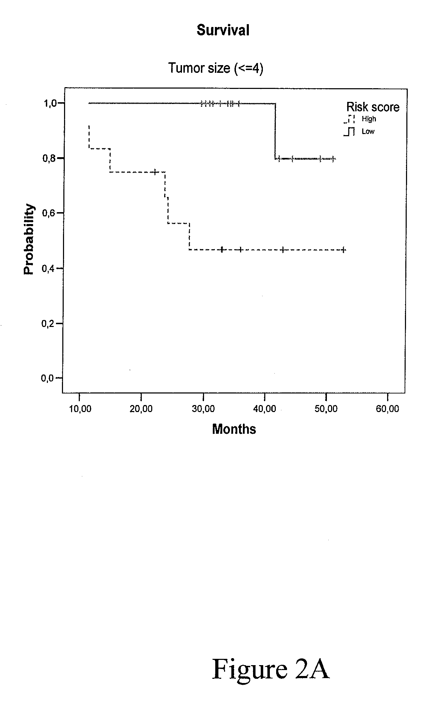

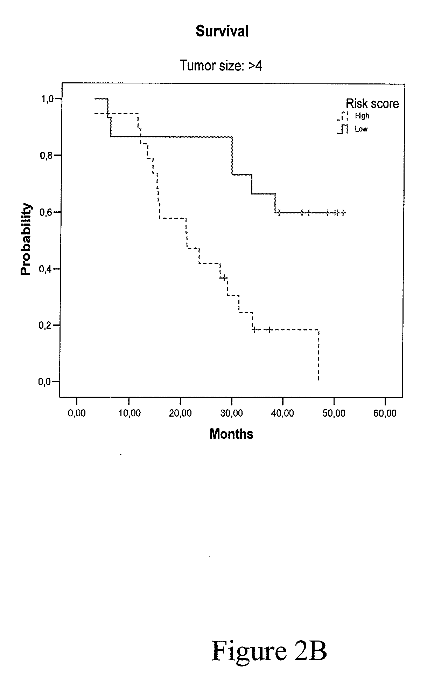

Median survival for high-risk patients was 24 months (95% CI, 17.1-30.9), while it was not reached in the low-risk group (p <0.00001) (Fig. 1). The risk score model including CSF-1, EGFR and CA IX predicts overall survival with 70% accuracy. This model also performed well in terms of distant metastases development prediction with sensitivity 64% and specificity 73%. Subsequently, survival analysis relative to risk score was done in the group of patients with tumours smaller 4 cm and larger tumours. In both instances, the risk score was able to predict prognosis with very high level of accuracy. In the population of patients with smaller tumours (Fig 2A), the patients with low risk score had exquisitely good prognosis (93% were classified correctly). The high risk patients in this group had a median survival of 27.5 months (p=0.005) (Fig. 2A). In patients with tumours larger than 4 cm, all of the 19 high risk patients were classified correctly, as none of them survived 5 years after resection. In this group the median survival for low risk patients was not reached (p=0.002) (Fig. 2B). Interestingly, in the univariate analysis, overexpression of *EGFR, FN1, INR1, ANLN* and *KIAA0974* precisely predicts very poor prognosis (5-year survival < 20%). (Median survival curves according to these genes and for: *CSF1, PH4, VEGFC, ISNR* and *NTRK1* are provided in the supplementary materials). When the gene expression was correlated by p Spearman method biologically meaningful correlations between the genes were found. For example, the high levels of PH4 were related to low or no expression of CA IX (p = -0.33; p=0.007).

**Claims**

1. A method for predicting the survival of a patient suffering from non-small-cell lung cancer (NSCLC) comprising the steps of:

   a) isolating mRNA from a tissue, blood or plasma sample of the patient;
   b) determining the gene expression levels of EGFR, CSF-1 and CA IX genes in the sample;
   c) comparing the expression levels of EGFR, CSF-1 and CA IX genes in the sample with reference values for EGFR, CSF-1 and CA IX expression levels

   wherein an increased expression of the EGFR and CSF-1 genes and a decreased expression of the CA IX gene with respect to the reference values is indicative that the survival prognosis of said patient will be decreased with respect to patients wherein expression of the EGFR, CSF-1 and CA IX gene is not altered.

2. The method of claim 1 which further comprises
   detecting in step (b) the expression levels of one or more additional genes selected from the group of fibronectin 1 (*FN1*), insulin receptor 1 (*ISNR*), anillin *(ANLN),* prolyl hydrolase 4 (*PH4*), *VEGF-C, NTRK1* and *KIAA0974,*
   comparing in step (c) the expression levels of the additional gene or genes with reference values for the expression levels of said genes
   wherein an increased expression of any of said genes with respect to the reference value in combination with an increased expression EGFR, CSF-1 and a decreased expression of CA IX is indicative that the survival prognosis of said patient will be decreased with respect to patients wherein expression of the same genes is not altered.

3. A method for predicting the survival of a patient suffering from non-small-cell lung cancer (NSCLC) comprising the steps of

   a) isolating mRNA from a tissue, blood or plasma sample of the patient;
   b) determining the gene expression level of EGFR, CSF-1 and CA IX genes in the sample;
   (c) calculating a risk score on the basis of the expression levels of EGFR, CSF-1 and CA IX genes using the formula

   $[0,93 \text{x} CSF + 1,4 \text{x} CA\ IX + 1,1\ \text{x}\ EGFR]$ wherein CSF, CA IX and EGFR are the z-scores of the expression levels of the CSF-1, CA IX and EGFR genes

   wherein a risk score higher than 0 is indicative of poor survival.

4. A method according to claims 1 to 3 wherein the NSCLC is squamous cell carcinoma.

5. A method according to claims 1 to 4 wherein the NSCLC patient is a stage IA, IB or stage II patient.

6. A method according to any of claims 1 to 5, wherein the nucleic acid is isolated from a tumour sample of the patient.

7. A method according to any of claims I to 6, wherein the nucleic acid is isolated from a blood or serum sample of the patient.

8. A method for deciding whether a patient suffering NSCLC should be treated with chemotherapy which comprises the steps of

   a) isolating mRNA from a tissue, blood or plasma sample of the patient;
   b) determining the gene expression level of EGFR, CSF-1 and CA IX genes in the sample;
   c) comparing the expression levels of EGFR, CSF-1 and CA IX genes in the sample with reference values for EGFR, CSF-1 and CA IX expression levels

   wherein an increased expression of the EGFR and CSF-1 genes and a decreased expression of the CA IX gene with respect to the reference values is indicative that the patient should be treated with chemotherapy.

9. A method for deciding whether a patient suffering NSCLC should be treated with chemotherapy comprising the steps of

   a) isolating mRNA from a tissue, blood or plasma sample of the patient;
   b) determining the gene expression level of EGFR, CSF-1 and CA IX genes in the sample;
   (c) calculating a risk score on the basis of the expression levels of EGFR, CSF-1 and CA IX genes using the formula

   $[0,93 \text{x} CSF + 1,4 \text{x} CA\ IX + 1,1\ \text{x}\ EGFR]$ wherein CSF, CA IX and EGFR are the z-scores of the expression levels of the CSF-1, CA IX and EGFR genes

   wherein a risk score higher than 0 is indicative that the patient should be treated with chemotherapy.

**10.** A method according to claims 8 or 9 wherein the NSCLC patient is a stage 1 or stage 11 patient.

**11.** A kit for detecting altered expression of the genes EGFR, CSF-1 and CA IX which comprises a first pair of primers which is capable of amplifying a region of EGFR, a second pair of primers which is capable of amplifying a region of CSF-1 and a third pair of primers which is capable of amplifying a region of the CA IX gene.

**12.** A kit according to claim 11 which further comprises at least a pair of primers capable of amplifying a region of a gene selected from the group of *FN1, ISNR, ANLN, PH4, VEGF-C, NTRK1* and *KIAA0974.*

**13.** A kit according to claim 11 or 12 which further comprises a reverse transcriptase.

**14.** Use of a kit as defined in claims 11 to 13 for predicting the survival of a patient suffering from NSCLC.

**15.** Use of a kit according to claims 11 to 14 for deciding whether a patient suffering NSCLC should be treated with chemotherapy.

Figure 1

**Survival**

Tumor size (<=4)

Figure 2A

**Survival**

Tumor size: >4

Figure 2B

**European Patent Office**

# EUROPEAN SEARCH REPORT

Application Number

EP 07 10 6177

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | US 2006/188981 A1 (HARRIS ADRIAN L [GB] ET AL) 24 August 2006 (2006-08-24) * abstract * | 1-15 | INV. C12Q1/68 G01N33/53 |
| A | JP 2005 304497 A (INASAWA JOJI; BML INC) 4 November 2005 (2005-11-04) * the whole document * | 1-15 | |
| A | SIMI LISA ET AL: "Quantitative analysis of carbonic anhydrase IX mRNA in human non-small cell lung cancer." LUNG CANCER (AMSTERDAM, NETHERLANDS) APR 2006, vol. 52, no. 1, April 2006 (2006-04), pages 59-66, XP002446790 ISSN: 0169-5002 * abstract * | 1-15 | |
| A | SWINSON D E B ET AL: "Coexpression of epidermal growth factor receptor with related factors is associated with a poor prognosis in non-small-cell lung cancer." BRITISH JOURNAL OF CANCER 4 OCT 2004, vol. 91, no. 7, 4 October 2004 (2004-10-04), pages 1301-1307, XP002446791 ISSN: 0007-0920 * abstract * | 1-15 | TECHNICAL FIELDS SEARCHED (IPC) C12Q G01N |

-/--

The present search report has been drawn up for all claims

| Place of search | Date of completion of the search | Examiner |
|---|---|---|
| Munich | 14 August 2007 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**European Patent Office**

## EUROPEAN SEARCH REPORT

Application Number

EP 07 10 6177

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | KAMINSKA JANINA ET AL: "Pretreatment serum levels of cytokines and cytokine receptors in patients with non-small cell lung cancer, and correlations with clinicopathological features and prognosis. M-CSF - an independent prognostic factor" ONCOLOGY, S. KARGER AG, BASEL, CH, vol. 70, no. 2, 2006, pages 115-125, XP009088000 ISSN: 0030-2414 | 1-10 | |
| A | * abstract * * page 117; table 2 * ----- | 11-15 | |

TECHNICAL FIELDS
SEARCHED     (IPC)

The present search report has been drawn up for all claims

| Place of search | Date of completion of the search | Examiner |
|---|---|---|
| Munich | 14 August 2007 | Celler, Jakub |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

............................................................................................

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 07 10 6177

This annex lists the patent family members relating to the patent documents cited in the above-mentioned European search report.
The members are as contained in the European Patent Office EDP file on
The European Patent Office is in no way liable for these particulars which are merely given for the purpose of information.

14-08-2007

| Patent document cited in search report | Publication date | Patent family member(s) | Publication date |
|---|---|---|---|
| US 2006188981 A1 | 24-08-2006 | NONE | |
| JP 2005304497 A | 04-11-2005 | NONE | |

EPO FORM P0459

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1381691 A

- EP 1381681 A

### Non-patent literature cited in the description

- **JEON Y-K. et al.** *Lung Cancer.,* December 2006, vol. 54 (3), 387-98
- **HIRSCH, F.R. et al.** *J Clin Oncol.,* 15 October 2003, vol. 21 (20), 3798-807
- **CHEN JJ et al.** *J Clin Oncol.,* 2005, vol. 23, 953-64
- **UEMURA,Y. et al.** *Int.J.Cancer,* 2004, vol. 109, 826-832
- **MROCZKO et al.** *Pol Arch Med Wewn.,* March 2001, vol. 105 (3), 203-9
- **BUI et al.** *Clin Cancer Res,* 2003, vol. 9, 802-11
- **SIMI, L. et al.** *Lung Cancer.,* April 2006, vol. 52 (1), 59-66
- **KON-NO, H. et al.** *Lung Cancer.,* December 2006, vol. 54 (3), 409-18
- **GREINER J et al.** *Blood,* 2006, vol. 108, 4109-17

- **BUI, M.H. et al.** *Clin.Cancer Res.,* 2003, vol. 9, 802-811
- **RAPONI,M. et al.** *Cancer Res.,* 2006, vol. 66, 7466-7472
- **CHEN,J.J. et al.** *J.Clin.Oncol.,* 2005, vol. 23, 953-964
- **INAMURA,K. et al.** *Oncogene,* 2005, vol. 24, 7105-7113
- **POTTI, A. et al.** *N.Engl.J.Med.,* 2006, vol. 355, 570-580
- **CHEN et al.** *NEJM,* 2007, vol. 356, 11-19
- **ENDOH.** *J Clin Oncol.,* 2004, vol. 22, 811-9
- **CHEN et al.** *J Clin Oncol.,* 2005, vol. 23, 953-64
- **MULLER-TIDOW, C et al.** *Cancer Res,* 2005, vol. 65, 1778-82
- **SAMBROOK ; FISCHER ; MANIATIS.** Molecular Cloning, a laboratory manual. Cold Spring Harbor Laboratory Press, 1989